# EUROPEAN PATENT APPLICATION

(11) **EP 4 471 158 A1**
(43) Date of publication of application: **04.12.2024**
(21) Application number: 23747272.5
(22) Date of filing: 20.01.2023
(51) Int. Cl.: C12Q 1/6811, C12Q 1/6806, C12N 15/11, C12N 15/10

(54) **MRNA FOR PROTEIN EXPRESSION AND TEMPLATE THEREFOR**

(30) Priority: 27.01.2022 KR 20220012551
(71) Applicant: SK Bioscience Co., Ltd., Bundang-gu, Seongnam-si, Gyeonggi-do, 13494 (KR)
(72) Inventor: JEUNG, Woon-hee, Seongnam-si, Gyeonggi-do 13494 (KR); KIM, Jung-in, Seongnam-si, Gyeonggi-do 13494 (KR); SEO, Ki-Weon, Seongnam-si, Gyeonggi-do 13494 (KR)
(74) Representative: Vossius & Partner Patentanwälte Rechtsanwälte mbB
(86) International application number: PCT/KR2023/001035
(87) International publication number: WO 2023/146230

(57) **Abstract**

Provided are mRNA for protein expression and a template therefor, particularly, provided are an mRNA transcription vector comprising a gene construct according to one embodiment, a method of preparing an mRNA molecule, the method comprising the step of performing transcription using the mRNA transcription vector, an mRNA molecule prepared by the method, and a pharmaceutical composition comprising the mRNA molecule as an active ingredient.

## Description

### [Technical Field]

The present disclosure relates to mRNA for protein expression and a template therefor.

### [Background Art]

Recently, mRNA has been attracting attention as an active pharmaceutical ingredient (API). There are three main advantages of vaccines using mRNA. First, in terms of production, the process is relatively simple, they may be prepared in a short period of time, and allow for a rapid and flexible response to emergencies such as pandemics or to mutations. Second, in terms of function, when intracellular delivery is effective, it is possible to induce both systemic immunity and cellular immunity, and it is expected to prevent diseases more effectively than other types of vaccines. Third, in terms of safety, there are fewer side effects, and unlike DNA, RNA has a great advantage of not causing modification of human genes by entering the nucleus. Accordingly, there is a demand for the development of appropriate expression systems for the mass-production of mRNA.

### [Disclosure]

### [Technical Problem]

The present disclosure relates to mRNA for protein expression and a template therefor.

### [Technical Solution]

An object of the present disclosure is to provide an mRNA transcription vector comprising a promoter region recognized by RNA polymerase and a gene construct operably linked to the promoter region.

Another object of the present disclosure is to provide a method of preparing an mRNA molecule, the method comprising the step of performing transcription using the mRNA transcription vector as a template.

Still another object of the present disclosure is to provide an mRNA molecule prepared above.

Still another object of the present disclosure is to provide a pharmaceutical composition comprising the prepared mRNA molecule as an active ingredient.

Still another object of the present disclosure is to provide use of the mRNA transcription vector in the preparation of mRNA and in the preparation of the pharmaceutical composition.

### [Advantageous Effects]

Since the expression level of a target protein may be increased by an mRNA transcription vector comprising a specific UTR combination of the present disclosure, the target protein may be expressed in large quantities.

### [Brief Description of the Drawing]

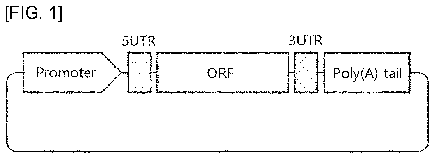

FIG. 1 illustrates an mRNA transcription vector prepared in Example 1.

### [Detailed Description of Preferred Embodiments]

An aspect of the present disclosure provides an mRNA transcription vector.

The mRNA transcription vector comprises a promoter region recognized by RNA polymerase, and a gene construct operably linked to the promoter region.

In one specific embodiment, the gene construct is characterized by comprising
(1) a 5'-untranslated region (5'-UTR) region,
(2) an open reading frame (ORF) region comprising a nucleotide sequence encoding a target protein, which is operably linked to the 5'-UTR region, and
(3) a 3'-untranslated region (3'-UTR) region operably linked to the open reading frame region.

In another specific embodiment, the 3'-UTR region consists of SEQ ID NO: 1 or a nucleotide sequence having 90% or more sequence identity thereto, or the 5'-UTR region is characterized by consisting of SEQ ID NO: 47 or a nucleotide sequence having 90% or more sequence identity thereto.

The 3'-UTR region comprised in the mRNA expression vector according to any one of the preceding specific embodiments consists of SEQ ID NO: 1 or a nucleotide sequence having 90% or more sequence identity thereto, and the 5'-UTR region is characterized by being selected from SEQ ID NO: 5, 6, 7, 8, 9, 11, 12, 13, 14, 16, 19, 20, 21, 22, 23, 42 or a nucleotide sequence having 90% or more sequence identity thereto.

The 5'-UTR region comprised in the mRNA expression vector according to any one of the preceding specific embodiments consists of SEQ ID NO: 47 or a nucleotide sequence having 90% or more sequence identity thereto, or the 3'-UTR region is characterized by being selected from SEQ ID NO: 50, 51, 53, 54, 55, 60, 64, 65, 66, 67, 75, 91 or a nucleotide sequence having 90% or more sequence identity thereto.

The gene construct comprised in the mRNA expression vector according to any one of the preceding specific embodiments is characterized by comprising (1) a 5'-untranslated region (5'-UTR) region, (2) an open reading frame (ORF) region comprising a nucleotide sequence encoding a target protein, which is operably linked to the 5'-UTR region, and (3) a 3'-untranslated region (3'-UTR) region operably linked to the open reading frame region, wherein the 3'-UTR region consists of SEQ ID NO: 1 or a nucleotide sequence having 90% or more sequence identity thereto, or the 5'-UTR region consists of SEQ ID NO: 47 or a nucleotide sequence having 90% or more sequence identity thereto, when the 3'-UTR region consists of SEQ ID NO: 1 or a nucleotide sequence having 90% or more sequence identity thereto, the 5'-UTR region is selected from SEQ ID NO: 5, 6, 7, 8, 9, 11, 12, 13, 14, 16, 19, 20, 21, 22, 23, 42 or a nucleotide sequence having 90% or more sequence identity thereto, and when the 5'-UTR region consists of SEQ ID NO: 47 or a nucleotide sequence having 90% or more sequence identity thereto, the 3'-UTR region is selected from SEQ ID NO: 50, 51, 53, 54, 55, 60, 64, 65, 66, 67, 75, 91 or a nucleotide sequence having 90% or more sequence identity thereto.

The open reading frame region comprised in the mRNA expression vector according to any one of the preceding specific embodiments is characterized by comprising a nucleotide sequence encoding an antigen.

The open reading frame region comprised in the mRNA expression vector according to any one of the preceding specific embodiments is characterized by comprising a nucleotide sequence encoding an antigen derived from a pathogen.

The pathogen in the mRNA expression vector according to any one of the preceding specific embodiments is characterized by being selected from the group consisting of viruses, bacteria, prions, fungi, protozoa, viroids, and parasites.

The gene construct comprised in the mRNA expression vector according to any one of the preceding specific embodiments is characterized by further comprising a nucleotide sequence transcribed to 5' Cap, which is operably linked to the 5'-UTR.

The gene construct comprised in the mRNA expression vector according to any one of the preceding specific embodiments is characterized by further comprising a nucleotide sequence transcribed to poly A tail, which is operably linked to the 3'-UTR.

The poly A tail comprised in the mRNA expression vector according to any one of the preceding specific embodiments is characterized by comprising 20 adenines to 200 adenines.

The mRNA expression vector according to any one of the preceding specific embodiments is characterized by being a plasmid.

The mRNA expression vector according to any one of the preceding specific embodiments is characterized by being linearized.

Another aspect of the present disclosure provides a method of preparing an mRNA molecule.

In one specific embodiment, the method is characterized by comprising the step of performing transcription using the mRNA transcription vector of the preceding embodiment as a template.

In another specific embodiment, the method of preparing mRNA is characterized by comprising the step of performing *in vitro* transcription using the mRNA transcription vector of the preceding embodiment as a template.

Still another aspect of the present disclosure provides an mRNA molecule which is prepared by the method of preparing an mRNA molecule of the preceding embodiment.

Still another aspect of the present disclosure provides a pharmaceutical composition comprising the prepared mRNA molecule of the preceding embodiment as an active ingredient.

In one specific embodiment, the pharmaceutical composition is a pharmaceutical composition for preventing or treating a disease.

In another specific embodiment, the pharmaceutical composition is characterized by comprising a pharmaceutically acceptable excipient.

In a specific embodiment according to any one of the preceding specific embodiments, the pharmaceutical composition is an immunogenic composition.

The mRNA molecule comprised in the pharmaceutical composition according to any one of the preceding specific embodiments is characterized by forming a complex with at least one or more lipid components.

The mRNA molecule of the pharmaceutical composition according to any one of the preceding specific embodiments is characterized by forming a complex with at least one or more lipid components to form a lipidoid, a liposome, a lipid nanoparticle, and/or a lipoplex.

The pharmaceutical composition according to any one of the preceding specific embodiments is characterized by being administered intramuscularly or subcutaneously.

Still another aspect of the present disclosure provides a method of preparing a pharmaceutical composition comprising mRNA as an active ingredient.

In one specific embodiment, the method is characterized by comprising the step of obtaining mRNA by performing transcription using the mRNA transcription vector of the preceding embodiment as a template.

Still another aspect of the present disclosure provides use of the mRNA transcription vector in the preparation of mRNA.

Still another aspect of the present disclosure provides a composition for preparing the pharmaceutical composition comprising the mRNA transcription vector.

Still another aspect of the present disclosure provides use of the mRNA transcription vector in the preparation of a pharmaceutical composition.

Still another aspect of the present disclosure provides a gene construct comprising (1) a 5'-untranslated region (5'-UTR) region, (2) an open reading frame (ORF) region comprising a nucleotide sequence encoding a target protein, which is operably linked to the 5'-UTR region, and (3) a 3'-untranslated region (3'-UTR) region operably linked to the open reading frame region.

In one specific embodiment, the gene construct is characterized by comprising a 3'-UTR region consisting of SEQ ID NO: 1 or a nucleotide sequence having 90% or more sequence identity thereto; and a 5'-UTR region consisting of a nucleotide sequence selected from SEQ ID NO: 5, 6, 7, 8, 9, 11, 12, 13, 14, 16, 19, 20, 21, 22, 23, 42 or a nucleotide sequence having 90% or more sequence identity thereto.

In another specific embodiment, the gene construct is characterized by comprising a 5'-UTR region consisting of SEQ ID NO: 47 or a nucleotide sequence having 90% or more sequence identity thereto; and a 3'-UTR region consisting of a nucleotide sequence selected from SEQ ID NO: 50, 51, 53, 54, 55, 60, 64, 65, 66, 67, 75, 91 or a nucleotide sequence having 90% or more sequence identity thereto.

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

The "nucleotide sequence" of the present disclosure is also called "base sequence", and may also be called "polynucleotide" which is a DNA or RNA strand of a certain length or longer as a polymer of nucleotides in which nucleotide monomers are connected in a long chain form by covalent bonds.

The polynucleotide of the present disclosure may comprise modified nucleotides. In the present disclosure, the "modified nucleotide" refers to any non-typical nucleoside, nucleotide, or a corresponding phosphorylated form thereof. The modified nucleotide may have one or more backbone or base modifications. Examples of modified nucleotides may comprise dI, dU, 8-oxo-dG, dX and THF, and may comprise other natural or non-natural nucleotides known in the art.

In some embodiment of the present disclosure, uridine present in mRNA may exist as thymidine in each DNA encoding the mRNA. In addition, although a polynucleotide sequence presented in the present disclosure cites "T" in a representative DNA sequence, it may be recognized that "T" is replaced with "U" when the sequence represents RNA.

As used herein, the term "non-natural" refers to a polynucleotide, polypeptide, carbohydrate, lipid, or composition that does not exist in nature. Such a polynucleotide, polypeptide, carbohydrate, lipid, or composition may differ from natural polynucleotides polypeptides, carbohydrates, lipids, or compositions in one or more aspects. For example, a polymer (e.g., a polynucleotide, polypeptide, or carbohydrate) may differ in terms of the kind and arrangement of the component building blocks (e.g., nucleotide sequence, amino acid sequence, or sugar molecules). A polymer may differ from a natural polymer in terms of the molecule(s) to which it is linked.

As used herein, the term "operably linked" means a configuration in which a control sequence is placed at an appropriate position such that the control sequence directs transcription of a coding sequence. With respect to the objects of the present disclosure, it means that a nucleotide sequence is functionally linked so that mRNA transcription may occur.

As used herein, the term "mRNA transcription vector" refers to a vector comprising a template strand of a mRNA transcription.

For example, the vector of the present disclosure may comprise a promoter capable of initiating transcription, any operator sequence for controlling the transcription, a sequence encoding a target mRNA, and a sequence controlling termination of transcription and translation. For example, the vector of the present disclosure may comprise a restriction enzyme binding site for easy cloning.

For example, the promoter comprised in the vector of the present disclosure may be any promoter for any DNA-dependent RNA polymerase. The promoter may be used by selecting a promoter sequence with high binding affinity to RNA polymerase, but is not necessarily limited thereto.

The vector may be obtained by cloning a polynucleotide, e.g., cDNA, and introducing the same into an appropriate expression cassette or vector for transcription. DNA templates may be obtained by reverse transcription of mRNA or gene synthesis.

The vector of the present disclosure comprises a promoter region recognized by RNA polymerase, and a gene construct operably linked to the promoter region, and comprises
(1) a 5'-untranslated region (5'-UTR) region,
(2) an open reading frame (ORF) region comprising a nucleotide sequence encoding a target protein, which is operably linked to the 5'-UTR region, and
(3) a 3'-untranslated region (3'-UTR) region operably linked to the open reading frame region.

In the present disclosure, the "5'-UTR" refers to an untranslated region that exists upstream of the initiation codon. The 5'-UTR may be involved in regulating RNA translation and/or stabilizing mRNA.

The 5'-UTR sequence of the present disclosure may be modified or optimized to improve mRNA stability and/or translation accuracy. For example, avoiding gene sequences similar to the start region of ORF in the 5'-UTR may effectively prevent false starts of frames during mRNA translation. Further, some specific sequences may be added to the 5'-UTR in order to improve mRNA stability and translation accuracy. For example, it may be configured to more accurately start the translation process by inserting Kozak sequences.

In the present disclosure, the "3'-UTR" refers to an untranslated region that exists downstream of the mRNA coding region.

The 3'-UTR sequence of the present disclosure may be modified or optimized to improve mRNA stability and/or translation accuracy. For example, mRNA stability may be increased and half-life may be increased by using an appropriate sequence in the 3'-UTR.

The mRNA transcription vector of the present disclosure is characterized by comprising a specific combination of 5'-UTR and 3'-UTR.

In one embodiment, the mRNA transcription vector of the present disclosure comprises a 3'-UTR sequence comprising SEQ ID NO: 1 or a nucleotide sequence having 90% or more sequence identity thereto; and a 5'-UTR sequence comprising a nucleotide sequence selected from the group consisting of SEQ ID NO: 5, 6, 7, 8, 9, 11, 12, 13, 14, 16, 19, 20, 21, 22, 23, 42 or a nucleotide sequence having at least 90% or more sequence identity to any one sequence selected from the sequences.

In one embodiment, the mRNA transcription vector of the present disclosure comprises a 5'-UTR sequence comprising SEQ ID NO: 47 or a nucleotide sequence having 90% or more sequence identity thereto; and a 3'-UTR sequence comprising a nucleotide sequence selected from the group consisting of SEQ ID NO: 50, 51, 53, 54, 55, 60, 64, 65, 66, 67, 75, 91 or a nucleotide sequence having at least 90% or more sequence identity to any one sequence selected from the sequences.

The UTR sequence may comprise a nucleotide sequence represented by a specific SEQ ID NO., or may consist essentially of or consist of the nucleotide sequence. In some embodiment of the present disclosure, the 5'-UTR and 3'-UTR sequences consist of a nucleotide sequence represented by a specific SEQ ID NO., or a nucleotide sequence having 90% or more sequence identity thereto.

In one embodiment, the mRNA transcription vector of the present disclosure comprises a 3'-UTR sequence consisting of SEQ ID NO: 1 or a nucleotide sequence having 90% or more sequence identity thereto; and a 5'-UTR sequence consisting of a nucleotide sequence selected from the group consisting of SEQ ID NO: 5, 6, 7, 8, 9, 11, 12, 13, 14, 16, 19, 20, 21, 22, 23, 42 or a nucleotide sequence having at least 90% or more sequence identity to any one sequence selected from the sequences.

In one embodiment, the mRNA transcription vector of the present disclosure comprises a 5'-UTR sequence consisting of SEQ ID NO: 47 or a nucleotide sequence having 90% or more sequence identity thereto; and a 3'-UTR sequence consisting of a nucleotide sequence selected from the group consisting of SEQ ID NO: 50, 51, 53, 54, 55, 60, 64, 65, 66, 67, 75, 91 or a nucleotide sequence having at least 90% or more sequence identity to any one sequence selected from the sequences.

Further, a nucleotide sequence having homology and identity to a nucleotide sequence of a specific SEQ ID NO. and comprising deletion, modification, substitution, or addition of some sequences may also be comprised in the scope of the present disclosure as long as the sequence exhibits activity identical to that of the nucleotide sequence consisting of the SEQ ID NO.

The term "homology" or "identity" refers to a degree of relevance between sequences corresponding to each other in two given amino acid sequences or nucleotide sequences, and may be expressed as a percentage. The terms homology and identity may often be used interchangeably with each other.

Calculation of sequence identity may be performed by, for example, aligning two sequences for optimal comparison purposes (e.g., a gap is introduced into one or both of first and second nucleotide sequences for optimal alignment, and non-identical sequences may be ignored for comparison purposes). In certain embodiments, the length of a sequence aligned for comparison purposes is at least 30%, at least 40%, at least 50%, at least 60%, at least 70%, at least 80%, at least 90%, at least 95% or 100% of the length of the reference sequence. Subsequently, the nucleotides at corresponding nucleotide positions are compared. Tools for such alignment comprise those of the BLAST suite (Stephen F. Altschul, et al (1997), "Gapped BLAST and PSI-BLAST: a new generation of protein database search programs", Nucleic Acids Res. 25:3389-3402). Another popular local alignment technique is based on the Smith-Waterman algorithm (Smith, T.F. & Waterman, M.S. (1981) "Identification of common molecular subsequences." J. Mol. Biol. 147:195-197). A general global alignment technique based on dynamic programming is the Needleman-Wunsch algorithm (Needleman, S.B. & Wunsch, C.D. (1970) "A general method applicable to the search for similarities in the amino acid sequences of two proteins." J. Mol. Biol. 48:443-453.). According to those more recently known, a Fast Optimal Global Sequence Alignment Algorithm (FOGSAA) has been developed that purportedly produces global alignment of nucleotide and protein sequences faster than other optimal global alignment methods, comprising the Needleman-Wunsch algorithm.

The comparison of sequences and determination of percent identity between two sequences may be accomplished using a mathematical algorithm. For example, the percent identity between two nucleotide sequences may be determined using methods such as those described in the literature [Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991]. For example, the percent identity between two nucleotide sequences may be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences may, alternatively, be determined using the GAP program in the GCG software package using an NWSgapdna.CMP matrix. Methods commonly employed to determine percent identity between sequences comprise, but are not limited to, those disclosed in [Carillo, H., and Lipman, D., SIAM J Applied Math., 48:1073 (1988)]. Exemplary computer software to determine homology between two sequences comprises, but is not limited to, the GCG program package, a literature [Devereux, J., et al., Nucleic Acids Research, 12(1), 387 (1984)], BLASTP, BLASTN and FASTA Altschul, a literature [S. F. et al., J. Molec. Biol., 215, 403 (1990)].

In the present disclosure, the "open reading frame", also abbreviated as "ORF", refers to a segment or region of an mRNA molecule that encodes a target protein or polypeptide. The ORF comprises a continuous stretch of DNA, beginning with the initiation codon and ending with a stop codon, and is translated by the ribosome.

In one embodiment, the target protein or polypeptide may be an antigen.

In the present disclosure, the "antigen" refers to a substance that may be recognized by the immune system and may trigger an antigen-specific immune response. For example, the antigen may be recognized by the adaptive immune system and may trigger an immune response through the formation of antibodies and/or antigen-specific T cells as part of the adaptive immune response. For example, the antigen may comprise a peptide or protein that may be presented to T cells by MHC. For example, the antigen may be a peptide/protein fragment comprising at least one epitope (antigen determinant), or a variant or derivative thereof.

With respect to the objects of the present disclosure, the antigen may be a translation product of the above-mentioned mRNA.

For example, the antigen may be derived from a pathogen.

For example, the pathogen may be selected from the group consisting of viruses, bacteria, prions, fungi, protozoa, viroids, and parasites, but is not limited thereto.

For example, the antigen may be a peptide or protein antigen derived from a pathogen associated with an infectious disease.

For another example, the antigen of the present disclosure may be a tumor associated antigen. Examples of the tumor associated antigen may comprise tissue differentiation antigens, tumor germline antigens (e.g., NY-ESO-1 or MAGE-3), normal proteins overexpressed by tumor cells (e.g., EGFR, Muc-1, Her2/neu), oncovirus proteins (e.g., EBV, HPV), tumor-specific mutant antigen (e.g., Mum-1, β-Catenin or CDK4). These antigens may be personalized cancer antigens or tissue-specific antigens, but are not limited to the mentioned examples.

In another embodiment, the target protein may be a protein involved in preventing or treating a disease. For example, the protein may be selected from a chimeric antigen receptor (CAR) capable of recognizing a targeted antigen, an immunostimulatory cytokine polypeptide, and a telomerase reverse transcriptase. For another example, the protein may be related to the prevention or treatment of metabolic diseases, comprising obesity. Examples of such target proteins comprise, but are not limited to, fibroblast growth factor 21 (FGF21) and leptin.

In one embodiment, the mRNA transcription vector of the present disclosure may further comprise a nucleotide sequence transcribed to 5'Cap.

In the present disclosure, the "5' Cap" refers to a modified entity that caps the end of mRNA. The 5' cap may generally be formed by modification, for example, a guanine derivative. For example, the 5' cap may be linked to the 5'-terminus via a 5'->5' triphosphate linkage. For example, the 5' cap may be methylated.

For example, capping may be performed along with transcription by adding a cap analogue during *in vitro* transcription. This is also called co-transcriptional capping. In the co-transcription method, caps are incorporated into the RNA molecule simultaneously with transcription during the *in vitro* transcription process. For another example, capping may be performed through an enzymatic reaction after the *in vitro* transcription. In these enzymatic reactions, enzymes such as triphosphatase and guanyl transferase may be involved. However, the method is not limited thereto, and mRNA may be allowed to comprise 5'Cap using methods known in the art.

In one embodiment, the mRNA transcription vector of the present disclosure may comprise a poly A tail.

In the present disclosure, the "poly A tail" refers to a continuous or discontinuous sequence of adenylic acid residues typically located at the 3' end of an RNA molecule. The poly A tail may follow the mRNA 3'-UTR of the present disclosure. Such a poly A tail may consist of or may comprise 20 or more, 25 or more, 40 or more, 60 or more, 80 or more, or 100 or more, or about 200 or more adenyl (A) nucleotides. Based on the number of nucleotides in the poly-A tail, generally, at least 75%, at least 80%, at least 85%, at least 90%, at least 95%, at least 96%, at least 97%, at least 98%, or at least 99% thereof is A nucleotides, but the remaining nucleotides may be a nucleotide other than A nucleotide, for example, U nucleotide (uridylic acid), G nucleotide (guanylic acid), or C nucleotide (cytidylic acid). For example, the poly A tail may comprise a modification that delays degradation of mRNA. For example, the poly A tail may comprise 20 adenines to 200 adenines.

For example, the vector of the present disclosure may comprise a selection marker for the confirmation of transformation. Markers that confer selectable phenotypes such as drug resistance, auxotrophy, resistance to cytotoxic agents may be used. In an environment treated with a selective agent, only cells expressing the selection marker survive or exhibit other phenotypic traits, and thus transformed cells may be selected, but are not limited to the above-described examples.

The *"in vitro* transcription" of the present disclosure relates to a process by which RNA is synthesized in a cell-free system (*in vitro*)*.* In the method of the present disclosure, the vector of the present disclosure is used as a template for producing mRNA.

For example, the vector may be linearized before performing *in vitro* transcription. The linearized vector may be obtained by treating the vector with appropriate restriction enzymes.

For example, the vector of the present disclosure may be a plasmid. However, there is no particular limitation thereon, and vectors known in the art may be used.

As used herein, the term "pharmaceutical composition" refers to a composition which may be used for preventing or treating a disease.

As used herein, the term "immunogenicity" refers to the property of inducing an immune response when introduced into the body. A disease may be prevented by administering the immunogenic composition of the present disclosure to an individual. Accordingly, the "pharmaceutical composition for preventing a disease" of the present disclosure may be an "immunogenic composition" or a "vaccine composition." The pharmaceutical composition may further comprise pharmaceutically acceptable vehicles, excipients, binders, carriers, preservatives, buffers, isotonic agents, emulsifying agents, or wetting agents.

RNA comprised in the pharmaceutical composition of the present disclosure may be formulated using one or more excipients in order to increase stability; to increase cell transfection; to permit the sustained or delayed release (e.g., from a depot formulation); to alter the biodistribution (e.g., targeting to specific tissues or cell types); to increase the translation of encoded protein *in vivo*; and/or to alter the release profile of encoded protein (antigen) *in vivo.* Accordingly, the pharmaceutical composition of the present disclosure may comprise pharmaceutically acceptable excipients.

The pharmaceutical composition may be formulated. This formulation may be a solid or liquid, or a combination thereof.

For example, the mRNA comprised in the pharmaceutical composition of the present disclosure may be in a naked form, in a form of being comprised in a vector, or in a complex form with a delivery system.

For example, the pharmaceutical composition of the present disclosure may comprise a lipidoid, a liposome, a lipoplex, a lipid nanoparticle, a compound polymer, a peptide/protein, a cell, a nanoparticle mimic, or a nanotube as a delivery system for delivering mRNA. The mRNA and the delivery system may form a complex.

Examples of the polymer may comprise polyamidoamine (PAA), poly(beta-amino-ester) (PBAE), poly(ethylenimine) (PEI), poly(I-lysine) (PLL), spermine, chitosan and/or polyurethane, which may be used as an mRNA delivery system. Examples of the nanoparticle may comprise ferritin and platelet membrane-coated nanoparticles (PNP). Examples of the peptide/protein may comprise protamine.

For example, the lipid nanoparticle for delivering mRNA may comprise phospholipids, structural lipids, PEG lipids, ionizable lipids, and/or quaternary amine compounds.

For example, the pharmaceutical composition of the present disclosure may comprise an mRNA molecule complexed with at least one or more lipid components. For example, the mRNA molecule may form a liposome, lipid nanoparticle, and/or lipoplex complex with at least one or more lipid components.

For example, when the pharmaceutical composition of the present disclosure may be administered to a subject, mRNA may be translated *in vivo* to produce an antigenic polypeptide (antigen).

For example, the pharmaceutical composition of the present disclosure may be administered by infusion or injection, similar to the administration of vaccines known in the art, for example, via percutaneous, oral, parenteral routes, such as intradermal, subcutaneous, intravenous, intramuscular, intranodal and/or intraperitoneal injection, or intranasal (e.g., tracheal inhalation) route of administration. Further, the composition may be administered intranasally, vaginally, rectally, orally, or transdermally. For another example, the vaccine composition may be administered by a "needle-free" delivery system.

For example, the pharmaceutical composition of the present disclosure may be used to generate antibodies in mammals.

The pharmaceutical composition of the present disclosure may be provided in a single dose or a multidose dose. The "multiple dose" or "multi-dose" refers to a unit of formulation comprising a dose which may be administered more than once (e.g., twice or more) to one subject or more than one subject.

The immunogenic composition of the present disclosure may comprise an immunologically effective amount of polynucleotide. The term "immunologically effective amount" refers to the amount effective in inducing an antibody reaction against an antigen when administered to an individual. The immunologically effective amount may vary depending on the health and physical conditions of an individual being treated, the individual's age, the capacity of the individual's immune system to synthesize antibodies, the level of protection required, the formulation of the composition, the physician's assessment of the medical situation, and other related factors.

The immunogenic composition provided in the present disclosure may comprise an adjuvant. In the present disclosure, the "adjuvant" refers to a substance used to increase the immunogenicity of the immunogenic composition of the present disclosure. The adjuvant is often given to enhance the immune response.

The composition for preparing the pharmaceutical composition of the present disclosure may comprise components required for synthesizing RNA. Specifically, components for *in vitro* transcription (IVT) may be comprised.

For example, the composition may comprise RNA polymerase. For example, the composition may comprise ATP, GTP, UTP, CTP or analogues thereof. For example, the composition may comprise a buffer solution.

In the method of preparing the pharmaceutical composition of the present disclosure, mRNA transcription may be performed *in vitro.* The *in vitro* transcription is as described above.

### [Mode for Carrying Out the Invention]

Hereinafter, the present disclosure will be described in more detail with reference to Examples and Experimental Examples. However, these Examples and Experimental Examples are only for illustrating the present disclosure, and the scope of the present disclosure is not intended to be limited by these Examples and Experimental Examples.

### Example 1. Construction of mRNA transcription vector and mRNA transcript transcribed therefrom

In this Example, mRNA transcription vectors consisting of various combinations of 5'/3'-untranslated region (UTR) sequences and mRNA transcripts transcribed therefrom were constructed. In this Example, to easily examine the expression level of a target protein, d2EGFP was used as an example of the target protein.

### 1-1. Construction of mRNA transcription vector

As shown in FIG. 1, an mRNA transcription vector was constructed using, as a basic framework, a structure in which a promoter region sequence, a 5'-UTR region sequence, an ORF region sequence, a 3'-UTR region sequence, and a poly A-tail region sequence were operably linked sequentially from the 5'-end to the 3'-end.

In more detail, the plasmid was constructed by inserting the 5'-UTR region sequence (TAATACGACTCACTATA), Kozak sequence (GCCACC), d2EGFP gene, 3'-UTR region sequence, poly A sequence, and Esp31 recognition site after the T7 promoter sequence using a pcDNA3.1 plasmid.

In this regard, several mRNA transcription vectors were prepared by varying only the UTR sequence,
sequence information, in which only the 5'-UTR was changed while the 3'-UTR (α-globin UTR used in the Moderna vaccine) was fixed, is shown in Table 1; and Table 2 to Table 4,
sequence information, in which only the 3'-UTR was changed while the 5'-UTR (α-globin UTR used in the Pfizer/BioNTech vaccine) was fixed, is shown in Tale 5; and Table 6 to Table 12, and
sequence information that was unchanged in all Examples is shown in Table 13.

Sequence information of Examples, in which only the 5'-UTR was changed while the 3'-UTR (α-globin UTR used in the Moderna vaccine) was fixed:

**[Table 1]**

| 3'-UTR (α-globin UTR used in Moderna vaccine) fixed sequence | | | | |
|---|---|---|---|---|
| **No.** | **Origin classification** | **Gene** | **3'-UTR sequence** | **SEQ ID NO.** |
| Moderna | - | α-globin | GCTGGAGCCTCGGTGGCCATGCTTCTTGCCC CTTGGGCCTCCCCCCAGCCCCTCCTCCCCTT CCTGCACCCGTACCCCCGTGGTCTTTGAATAA AGTCTGAGTGGGCGGCA | 1 |

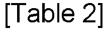

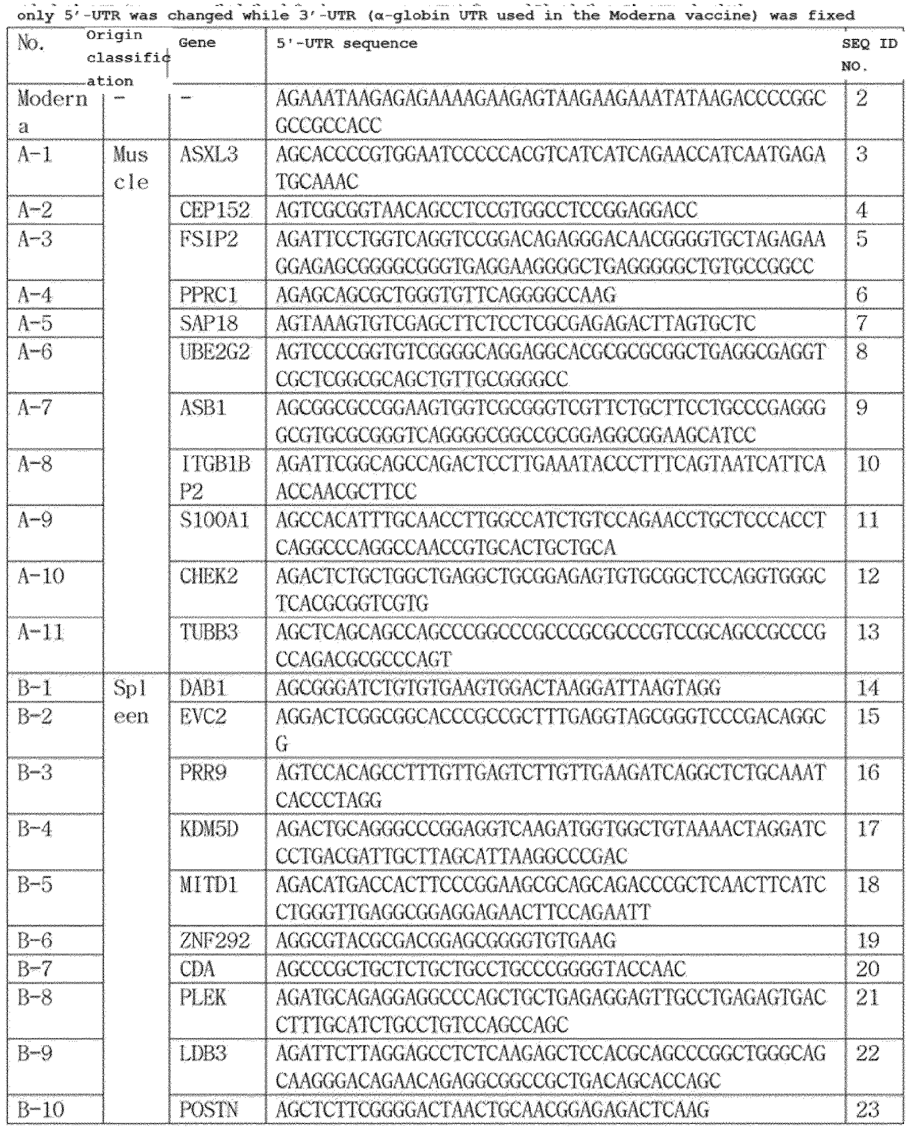

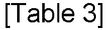

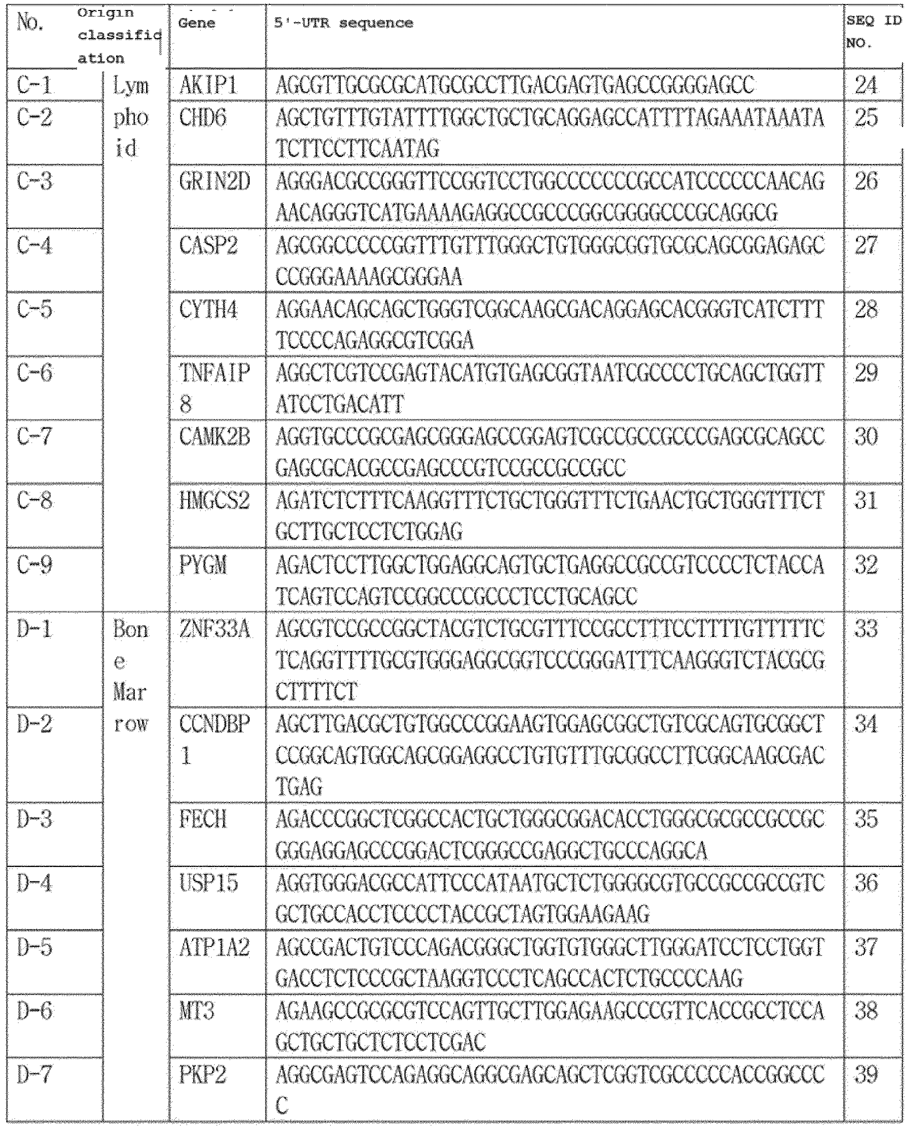

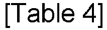

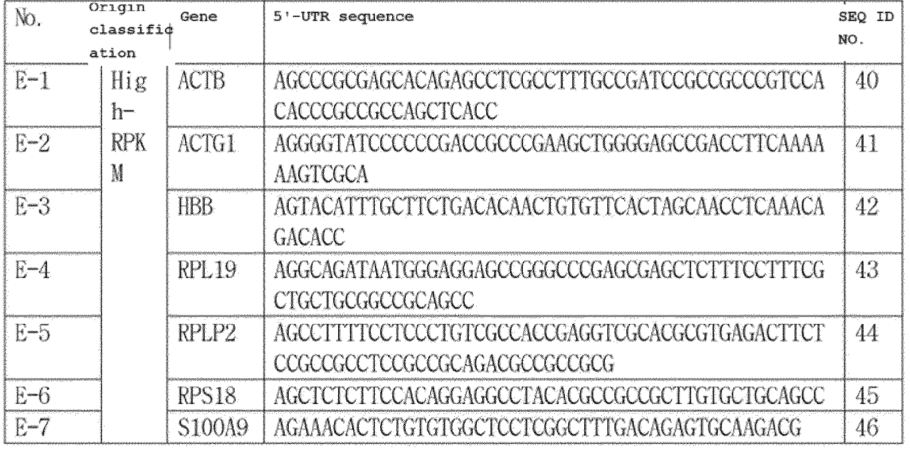

Sequence information of Examples, in which only the 3'-UTR was changed while the 5'-UTR (α-globin UTR used in the Pfizer/BioNTech vaccine) was fixed:

**[Table 5]**

| 5'-UTR (α-globin UTR used in Pfizer/BioNTech vaccine) fixed sequence | | | | |
|---|---|---|---|---|
| **No.** | **Origin classification** | **Gene** | **5'UTR sequence** | **SEQ ID NO.** |
| Pfizer/Bi oNTech | - | α-globin | AGAATAAACTAGTATTCTTCTGGTCCCCAC AGACTCAGAGAGAACCCGCCACC | 47 |

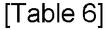

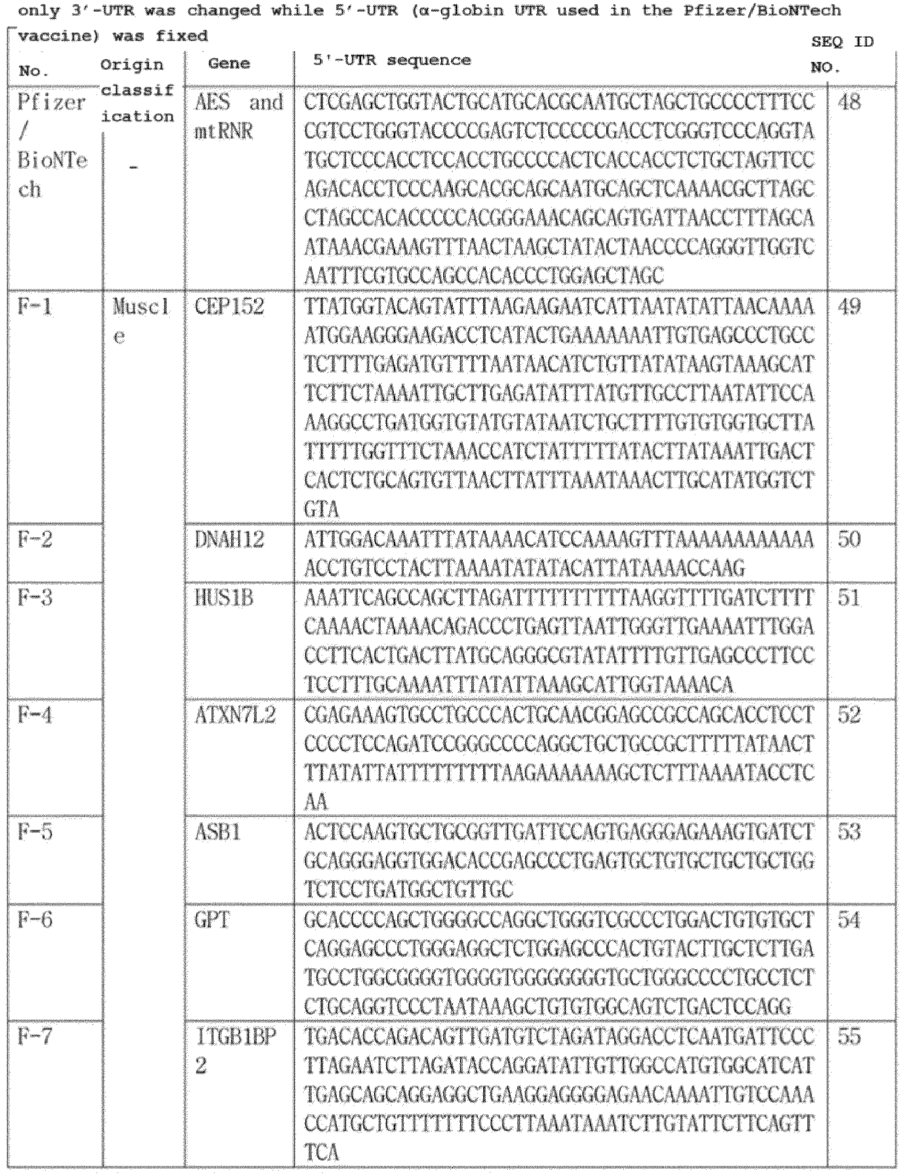

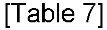

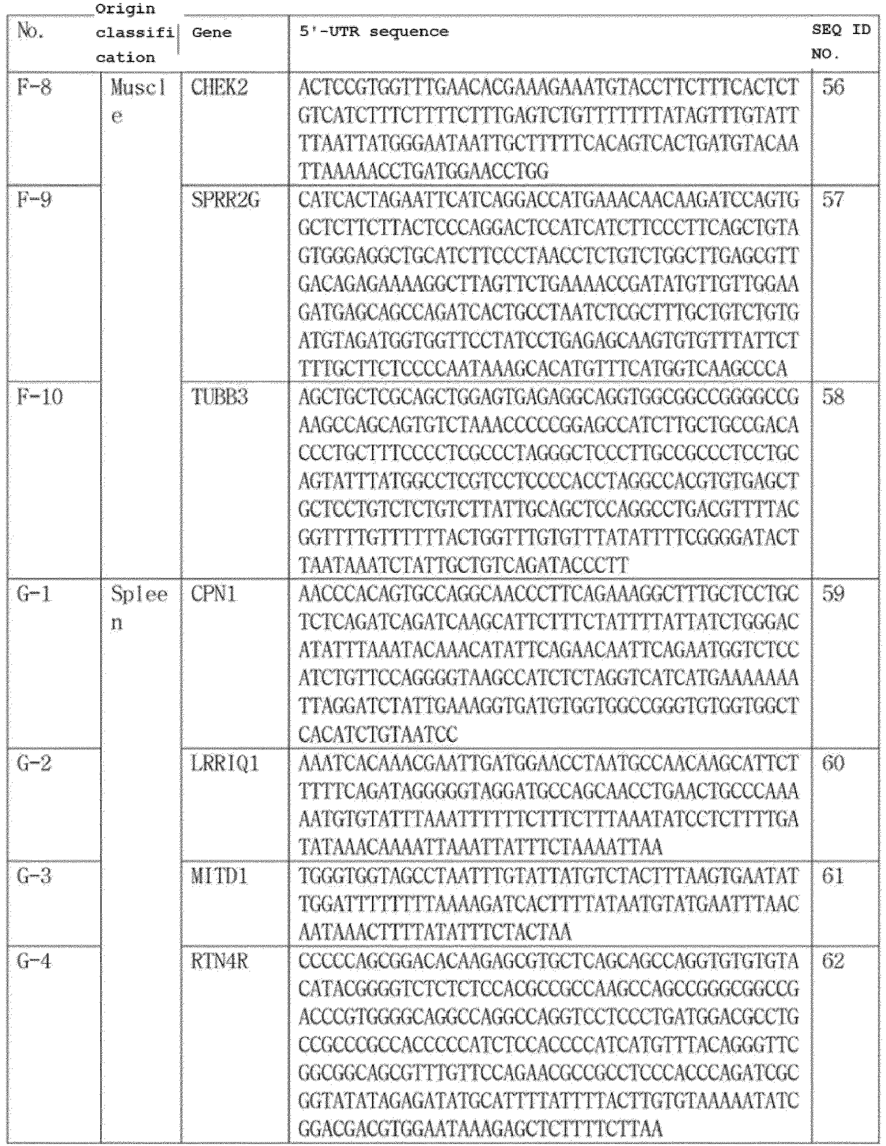

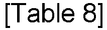

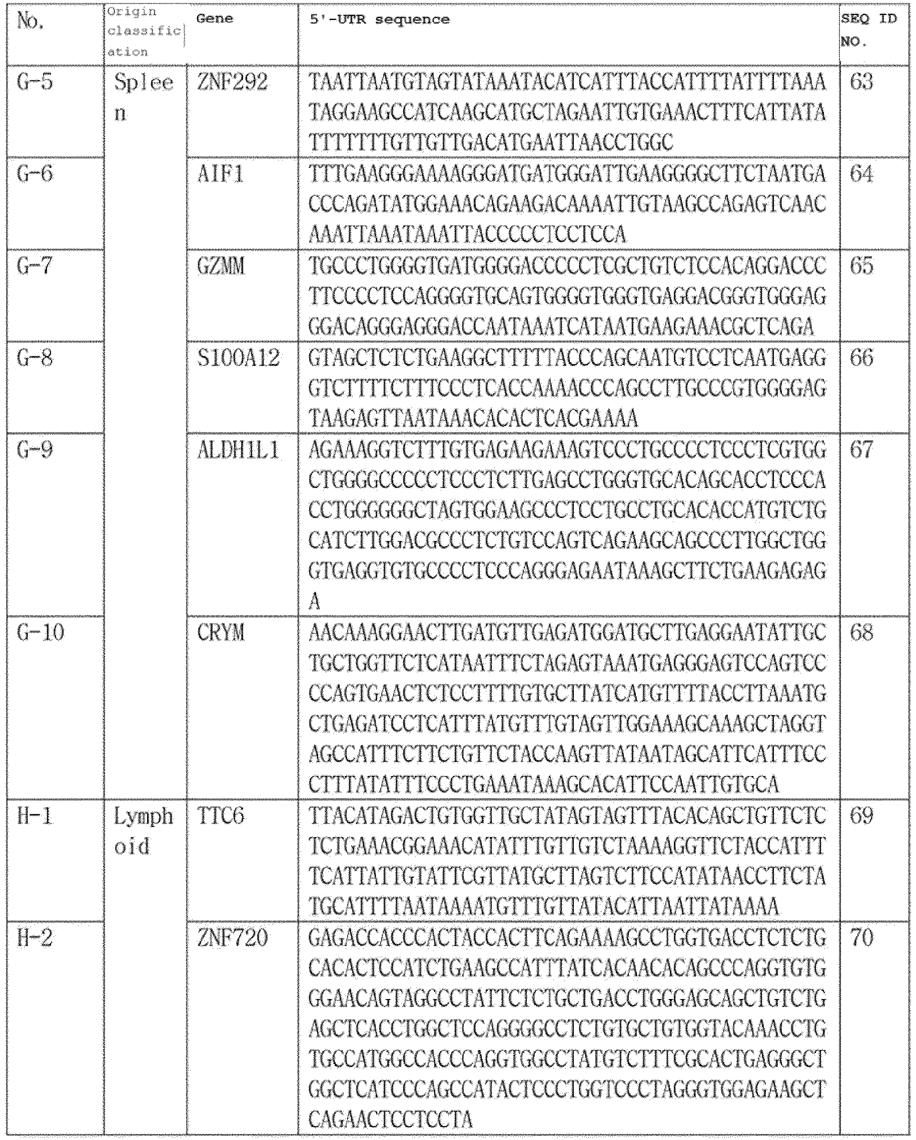

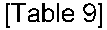

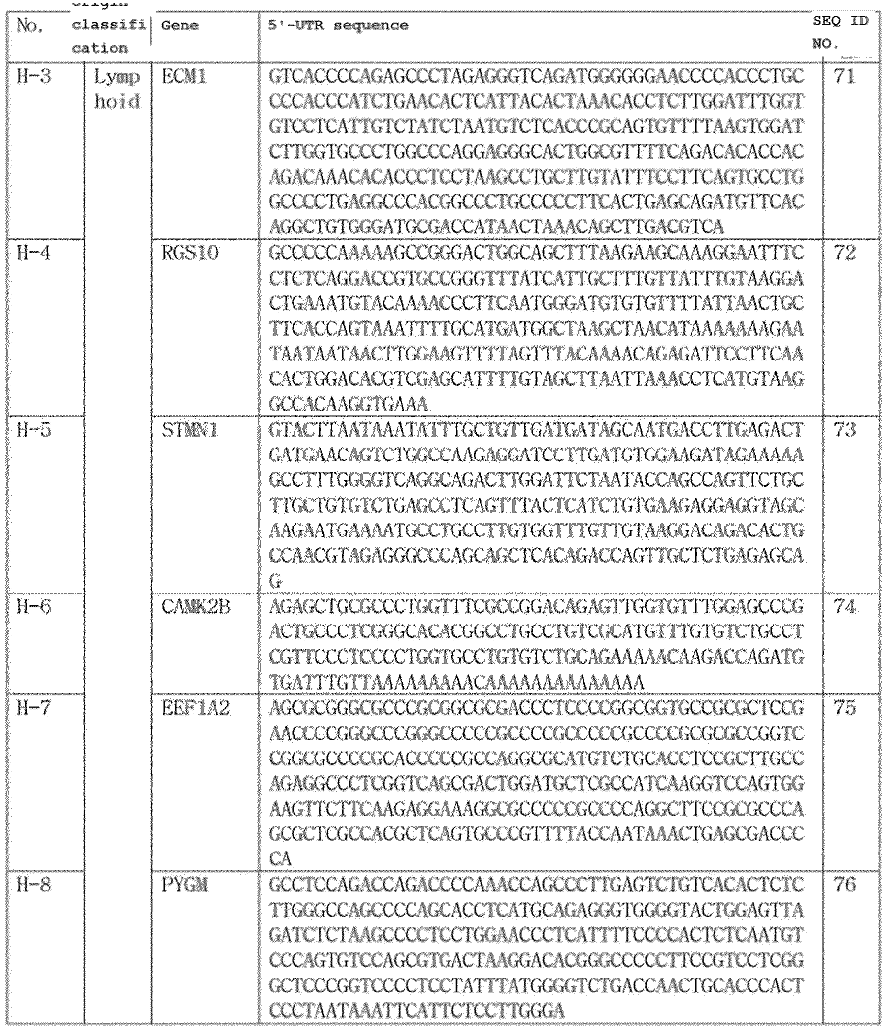

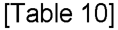

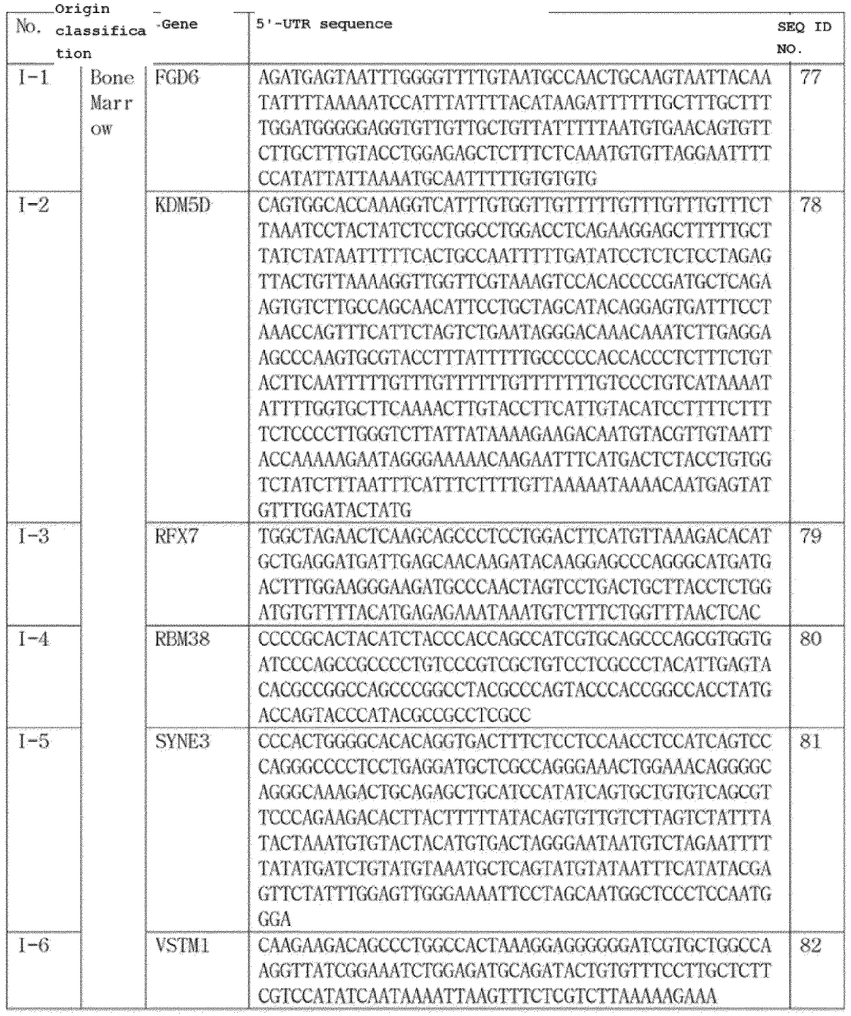

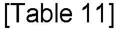

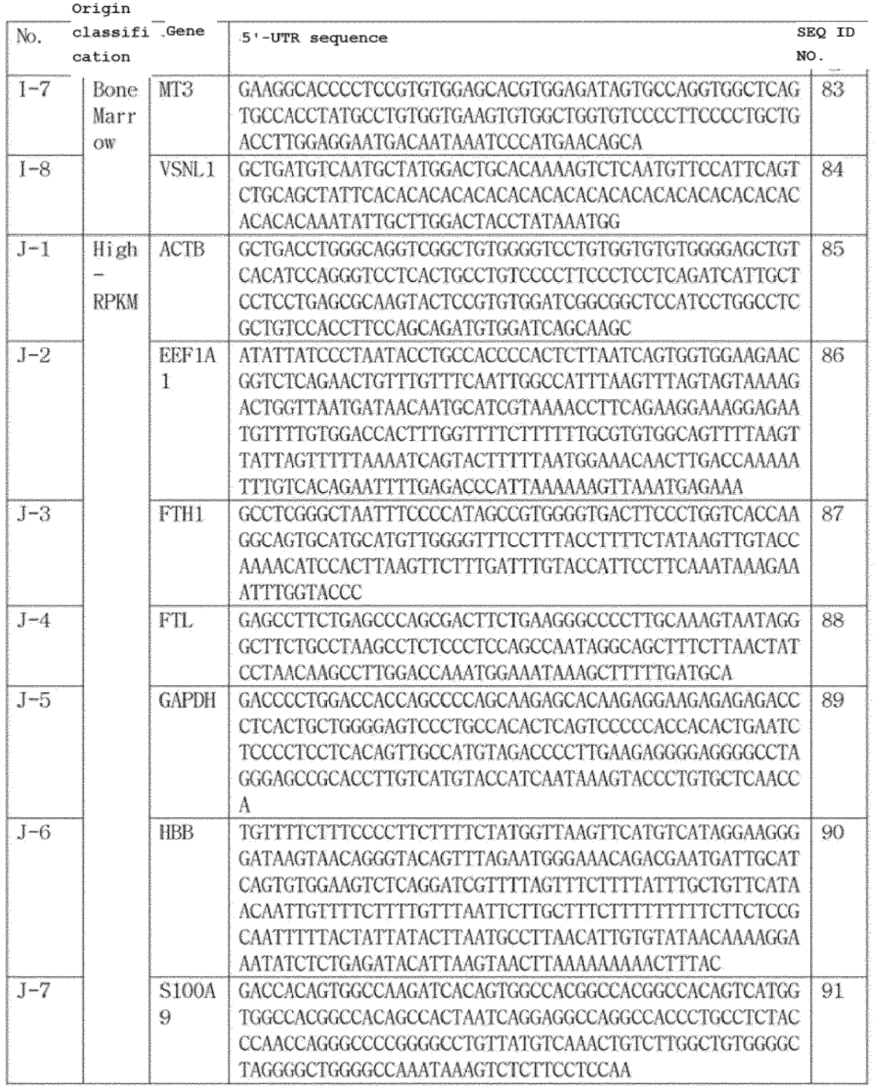

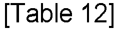

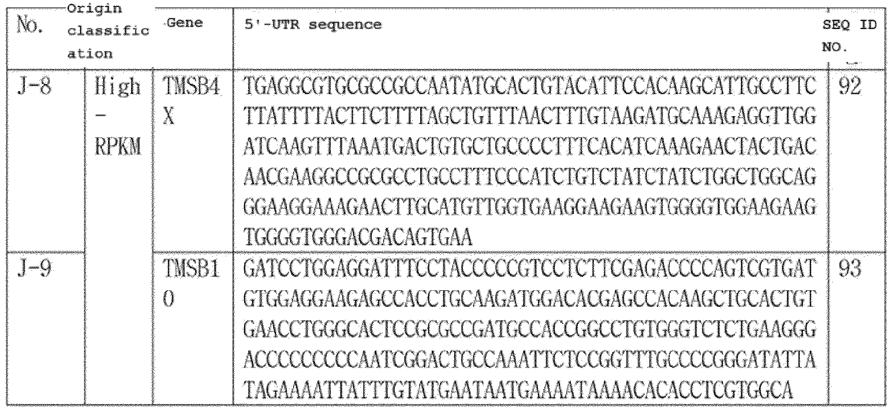

Sequence information unchanged in all Examples:

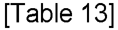

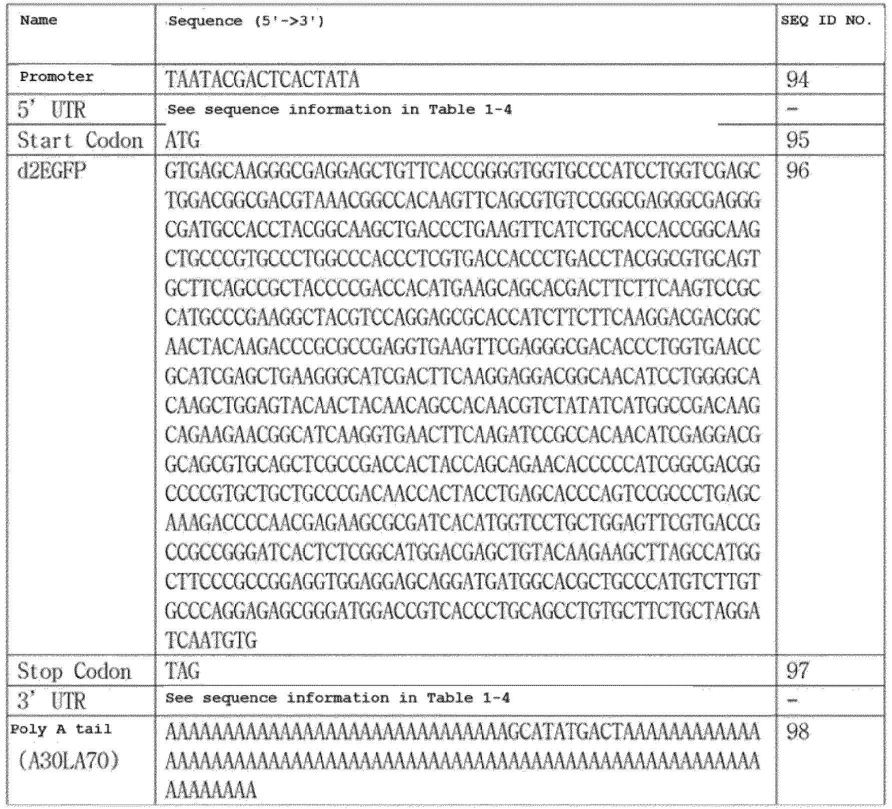

In total, 91 mRNA transcription vectors were constructed (specifically, Moderna Ref; A-1 to A-11; B-1 to B-10; C-1 to C-9; D-1 to D-7; E-1 to E-7; Pfizer/BioNTech Ref; F-1 to F-10; G-1 to G-10; H-1 to H-8; I-1 to I-8; J-1 to J-9).

### 1-2. Preparation of mRNA transcripts from mRNA transcription vectors

*E. coli* transformed with the prepared mRNA transcription vector plasmid was cultured, the plasmid was isolated using a CompactPrep Plasmid Midi Kit (Qiagen), and then the plasmid was linearized using Esp31 restriction enzyme. The linearized plasmid was recovered using a MinElute Reaction Cleanup Kit (Qiagen), and then used in an *in vitro* transcription (IVT) reaction for mRNA preparation. For this purpose, the linearized plasmid DNA template was mixed according to conditions shown in Table 14 below, and allowed to react at 37°C for 2 hours.

**[Table 14]**

| **Name of substance** | **Amount of use** |
|---|---|
| Linearized plasmid DNA template (total 1 ug) | 1 ug/40 ul |
| Nuclease-free H₂O | Use the amount to meet a total of 40 ul |
| ATP | 2 ul (concentration: 5 mM) |
| CTP | 2 ul (concentration: 5 mM) |
| UTP | 2 ul (concentration: 5 mM) |
| GTP | 2 ul (concentration: 5 mM) |
| CleanCap AG (Catalog No: N-7413) | 1.5 ul (concentration: 4 mM) |
| T7 RNA Polymerase mix | 2 ul |
| 10X Reaction buffer | 2 ul (0.5X) |
| Total | 40 ul |

After completing the above reaction, 2 units of DNase1 were added to each sample and allowed to react at 37°C for 15 minutes to remove the remaining linearized plasmid DNA template.

Thereafter, 25 ul of LiCl was mixed per 50 ul of RNA, and allowed to react at -20°C for 30 minutes, and RNA was precipitated by centrifugation. The RNA precipitate was washed with 70% ethanol, the remaining ethanol was removed, and then eluted with nuclease-free H₂O. After treatment at 65°C for 5 minutes to remove all remaining precipitate, an mRNA transcript suitable for transfection was prepared.

### Example 2. Evaluation of protein expression levels according to combination of UTR sequence

In this example, differences in protein expression levels according to various combinations of 5'/3'-untranslated region (UTR) sequences were evaluated.

A total of 91 mRNA transcripts prepared in Example 1 were independently transfected into primary skeletal muscle cells (ATCC, PCS-950-010) using Lipofectamine^{™} MessengerMAX (Thermo Fisher). In detail, primary skeletal muscle cells were seeded in a 24-well plate at a density of 2 x 10⁴ cells/well, and cultured for attachment at 37°C, 5% CO₂ for 5 hours or longer. For transfection, 25 ul of OptiMEM (Gibco) per mRNA sample was mixed with 0.75 ul of Lipofectamine^{™} MessengerMAX to prepare a MessengerMax mixture that was incubated for 10 minutes at room temperature, and an mRNA mixture comprising 50 ng of RNA transcript in 25 ul of OptiMEM was prepared. 25 ul of Messenger Max mixture and 25 ul of mRNA mixture were mixed and incubated at room temperature for 5 minutes, and then 50 ul of the mixed solution was treated to primary skeletal muscle cells. 24 hours and 48 hours after the procedure was performed, the d2EGFP protein expression levels were evaluated through Cytation 7 (BioTek). In detail, the expression levels were measured using GFP channels (469, 525), and then the fluorescence of the area comprised within a circle with a radius of 3200 um from the center of the well was analyzed. The minimum value of fluorescence intensity was set to over 5000, and the size of the cells to be analyzed was set to 30 um to 200 um. Thereafter, the total fluorescence intensity was measured by multiplying the analyzed mean fluorescence value by the cell count. Evaluation was performed a total of 4 times to 6 times on each mRNA transcript, and the results were expressed as the mean value.

### 2-1. Evaluation of GFP protein expression level when only 5'-UTR was changed while 3'-UTR (α-globin UTR used in Moderna vaccine) was fixed

GFP protein expression levels, when the 3'-UTR was fixed and only the 5'-UTR was changed, are shown in Tables 15 and 16 below.

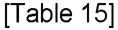

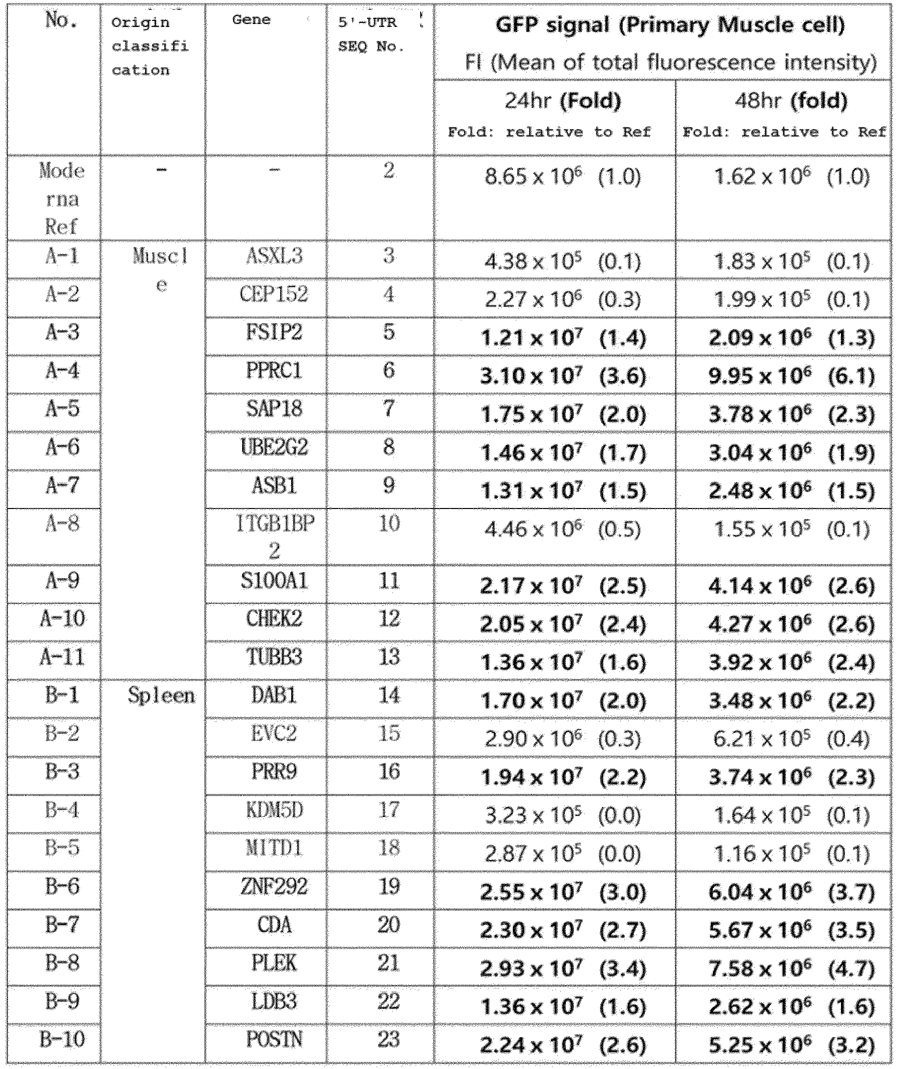

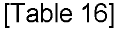

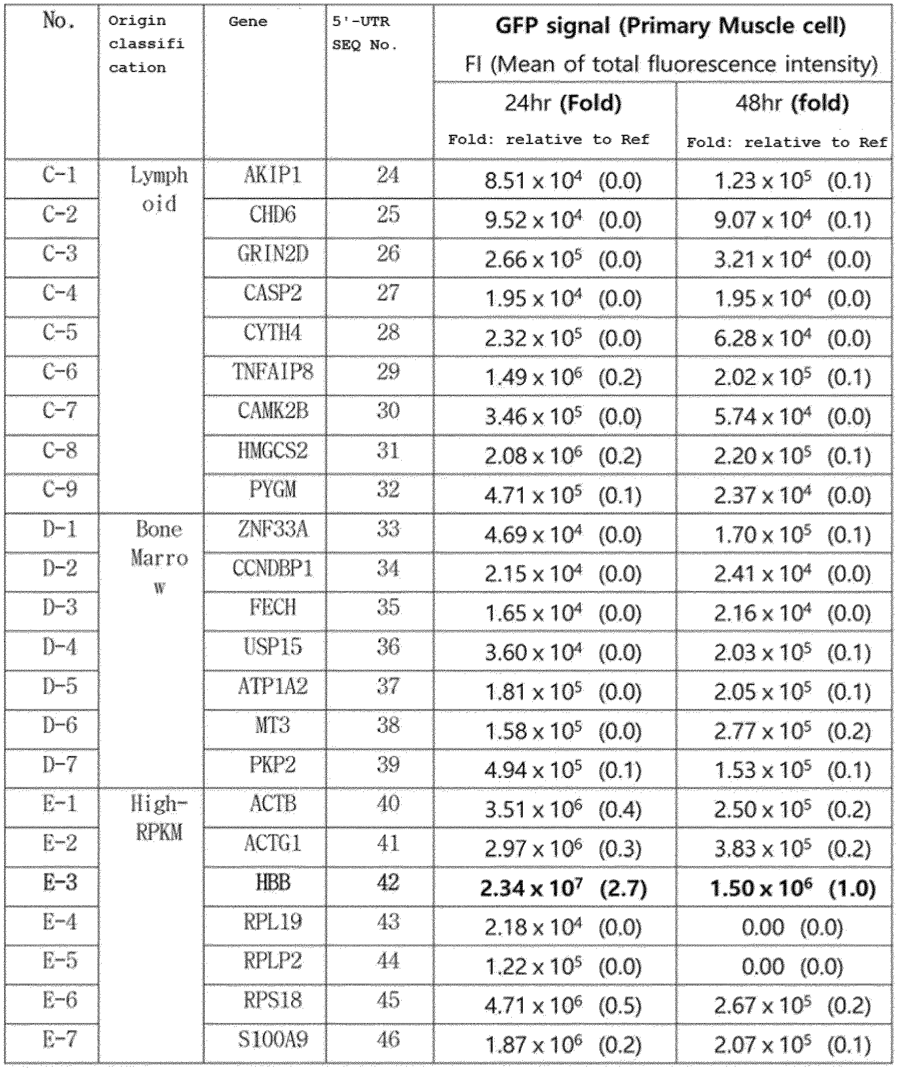

As shown in Tables 15 and 16, among the groups in which only 5'-UTR was changed while 3'-UTR (α-globin UTR used in Moderna vaccine) was fixed, A-3 to A-7 (SEQ ID NOS: 5 to 9), A-9 to A-11 (SEQ ID NOS: 11 to 13); B-1 (SEQ ID NO: 14), B-3 (SEQ ID NO: 16), B-6 to B-10 (SEQ ID NOS: 19 to 23); and E-3 (SEQ ID NO: 42) achieved very excellent protein expression efficiency, relative to Moderna Ref. and other same/different tissue-derived UTR combinations. In particular, the fold values in parentheses specifically indicate the superior degree of protein expression efficiency, relative to that of Moderna Ref.

### 2-2. Evaluation of GFP protein expression level when only 3'-UTR was changed while 5'-UTR (α-globin UTR used in Pfizer/BioNTech vaccine) was fixed

GFP protein expression levels, when the 5'-UTR was fixed and only the 3'-UTR was changed, are shown in Tables 17 and 18 below.

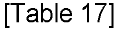

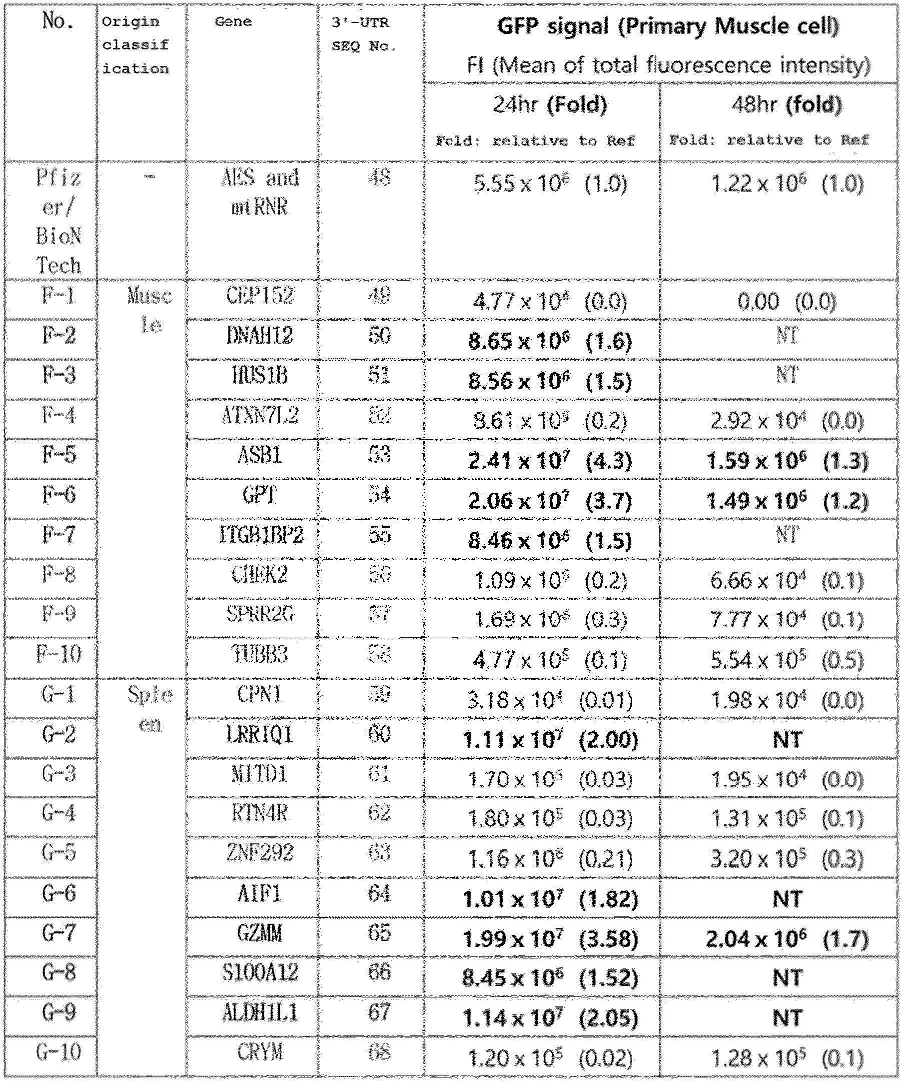

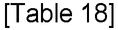

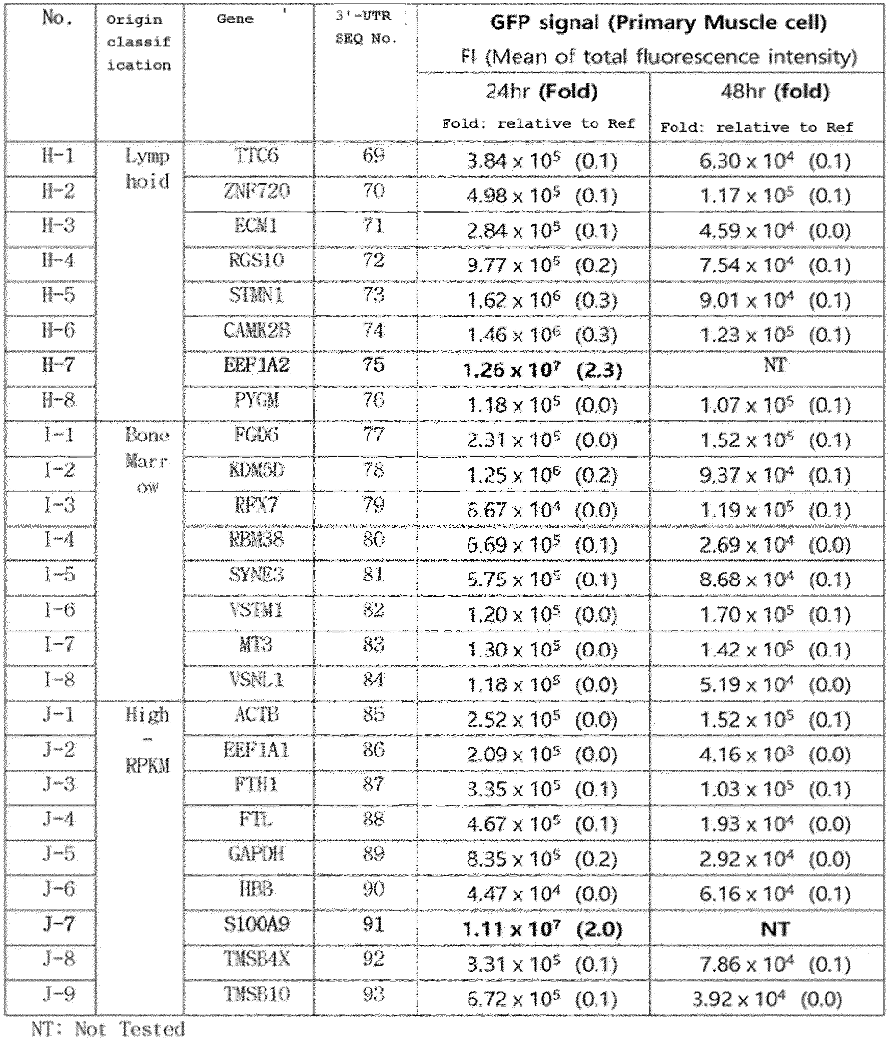

As shown in Tables 17 and 18, among the groups in which only the 3'-UTR was changed while 5'-UTR (α-globin UTR used in Pfizer/BioNTech vaccine was fixed, F-2 to F-3 (SEQ ID NOS: 50 to 51), F-5 to F-7 (SEQ ID NOS: 53 to 55); G-2 (SEQ ID NO: 60), G-6 to G-9 (SEQ ID NOS: 64 to 67); H-7 (SEQ ID NO: 75); and J-7 (SEQ ID NO: 91) achieved very excellent protein expression efficiency, relative to Pfizer/BioNTech Ref. and other same/different tissue-derived UTR combinations. In particular, the fold values in parentheses specifically indicate the superior degree of protein expression efficiency, relative to that of Pfizer/BioNTech Ref.

### 2-3. Conclusion

The results of 2-1 and 2-2 respectively confirmed that remarkably excellent protein expression efficiency was achieved in A-3 to A-7 (SEQ ID NOS: 5 to 9), A-9 to A-11 (SEQ ID NOS: 11 to 13); B-1 (SEQ ID NO: 14), B-3 (SEQ ID NO: 16), B-6 to B-10 (SEQ ID NOS: 19 to 23); and E-3 (SEQ ID NO: 42) among the groups in which only 5'-UTR was changed while 3'-UTR (α-globin UTR used in Moderna vaccine) was fixed; and F-2 to F-3 (SEQ ID NOS: 50 to 51), F-5 to F-7 (SEQ ID NOS: 53 to 55); G-2 (SEQ ID NO: 60), G-6 to G-9 (SEQ ID NOS: 64 to 67); H-7 (SEQ ID NO: 75); and J-7 (SEQ ID NO: 91) among the groups in which only 3'-UTR was changed while 5'-UTR (α-globin UTR used in Pfizer/BioNTech vaccine was fixed.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. An mRNA transcription vector comprising a promoter region recognized by RNA polymerase, and a gene construct operably linked to the promoter region,
wherein the gene construct comprises
(1) a 5'-untranslated region (5'-UTR) region,
(2) an open reading frame (ORF) region comprising a nucleotide sequence encoding a target protein, which is operably linked to the 5'-UTR region, and
(3) a 3'-untranslated region (3'-UTR) region operably linked to the open reading frame region,
wherein the 3'-UTR region consists of SEQ ID NO: 1 or a nucleotide sequence having 90% or more sequence identity thereto, or the 5'-UTR region consists of SEQ ID NO: 47 or a nucleotide sequence having 90% or more sequence identity thereto,
when the 3'-UTR region consists of SEQ ID NO: 1 or a nucleotide sequence having 90% or more sequence identity thereto, the 5'-UTR region is selected from SEQ ID NO: 5, 6, 7, 8, 9, 11, 12, 13, 14, 16, 19, 20, 21, 22, 23, 42 or a nucleotide sequence having 90% or more sequence identity thereto, and
when the 5'-UTR region consists of SEQ ID NO: 47 or a nucleotide sequence having 90% or more sequence identity thereto, the 3'-UTR region is selected from SEQ ID NO: 50, 51, 53, 54, 55, 60, 64, 65, 66, 67, 75, 91 or a nucleotide sequence having 90% or more sequence identity thereto.

2. The mRNA transcription vector of claim 1, wherein the open reading frame region comprises a nucleotide sequence encoding an antigen derived from a pathogen.

3. The mRNA transcription vector of claim 2, wherein the pathogen is selected from the group consisting of viruses, bacteria, prions, fungi, protozoa, viroids, and parasites.

4. The mRNA transcription vector of claim 1, wherein the gene construct further comprises a nucleotide sequence transcribed to 5' Cap, which is operably linked to the 5'-UTR.

5. The mRNA transcription vector of claim 1, wherein the gene construct further comprises a nucleotide sequence transcribed to a poly A tail, which is operably linked to the 3'-UTR region.

6. The mRNA transcription vector of claim 5, wherein the poly A tail comprises 20 adenines to 200 adenines.

7. The mRNA transcription vector of claim 1, wherein the mRNA transcription vector is a plasmid.

8. The mRNA transcription vector of claim 1, wherein the mRNA transcription vector is linearized.

9. A method of preparing an mRNA molecule, the method comprising the step of performing transcription using the mRNA transcription vector of any one of claims 1 to 8 as a template.

10. An mRNA molecule prepared by the method of claim 9.

11. A pharmaceutical composition comprising the mRNA molecule of claim 10 as an active ingredient.

12. The pharmaceutical composition of claim 11, wherein the mRNA molecule forms a complex with one or more lipid components to form any one selected from a liposome, a lipid nanoparticle, and a lipoplex.

13. The pharmaceutical composition of claim 11, wherein the pharmaceutical composition is administered intramuscularly or subcutaneously.

14. A gene construct comprising
(1) a 5'-untranslated region (5'-UTR) region,
(2) an open reading frame (ORF) region comprising a nucleotide sequence encoding a target protein, which is operably linked to the 5'-UTR region, and
(3) a 3'-untranslated region (3'-UTR) region operably linked to the open reading frame region,
wherein the 3'-UTR region consists of SEQ ID NO: 1 or a nucleotide sequence having 90% or more sequence identity thereto, or the 5'-UTR region consists of SEQ ID NO: 47 or a nucleotide sequence having 90% or more sequence identity thereto,
when the 3'-UTR region consists of SEQ ID NO: 1 or a nucleotide sequence having 90% or more sequence identity thereto, the 5'-UTR region is selected from SEQ ID NO: 5, 6, 7, 8, 9, 11, 12, 13, 14, 16, 19, 20, 21, 22, 23, 42 or a nucleotide sequence having 90% or more sequence identity thereto, and
when the 5'-UTR region consists of SEQ ID NO: 47 or a nucleotide sequence having 90% or more sequence identity thereto, the 3'-UTR region is selected from SEQ ID NO: 50, 51, 53, 54, 55, 60, 64, 65, 66, 67, 75, 91 or a nucleotide sequence having 90% or more sequence identity thereto.
